(19)

**Europäisches Patentamt**
**European Patent Office**
**Office européen des brevets**

(11) **EP 4 230 194 A1**

(12) **EUROPEAN PATENT APPLICATION**

(43) Date of publication:
**23.08.2023  Bulletin 2023/34**

(21) Application number: **23156810.6**

(22) Date of filing: **15.02.2023**

(51) International Patent Classification (IPC):
**A61K 9/16** *(2006.01)*    **A61K 9/10** *(2006.01)*
**A61K 38/00** *(2006.01)*    **A61P 5/02** *(2006.01)*
**A61K 47/38** *(2006.01)*

(52) Cooperative Patent Classification (CPC):
**A61K 9/1647; A61K 9/0019; A61K 9/10;**
**A61K 38/00; A61K 47/38; A61P 5/02**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority:  **18.02.2022  KR 20220021275**

(71) Applicant: **Inventage Lab Inc.**
**Gyeonggi-do 13403 (KR)**

(72) Inventors:
• **KIM, Ju Hee**
**Seongnam-si, Gyeonggi-do (KR)**
• **KIM, Min Sung**
**Seongnam-si, Gyeonggi-do (KR)**

(74) Representative: **BCKIP Part mbB**
**Siegfriedstraße 8**
**80803 München (DE)**

(54) **COMPOSITION FOR SUSTAINED-RELEASE INJECTION COMPRISING DESLORELIN**

(57)    The present disclosure, as a composition for sustained-release injection comprising deslorelin, can prevent initial over-release by adjusting the release rate of deslorelin at a target site and can exhibit the effect of deslorelin for 1 month or more by being exposed in an amount enough to show the effect of deslorelin.

Further, the microparticles can prepare microparticles that have a uniform particle size and are capable of exhibiting the effect of continuously releasing deslorelin for a long time.

EP 4 230 194 A1

Processed by Luminess, 75001 PARIS (FR)

## Description

### CROSS-REFERENCE TO RELATED APPLICATION(S)

This application claims priority to Korean Patent Application No. 10-2022-0021275, filed on February 18, 2022, the disclosure of which is incorporated herein by reference in its entirety.

### Technical Field

The present disclosure relates to a composition for sustained-release injection comprising deslorelin, and more particularly, to a composition for sustained-release injection comprising microparticles in which deslorelin is uniformly distributed.

### Description of the Related Art

Orchiectomy (removal of the testicles) or castration in male ruminants is necessary for a number of reasons, and is for primarily reducing aggression, reducing the risk of harm to humans and other animals, and facilitating handling.
Further, since orchiectomy (removal of the testicles) or castration in male ruminants is for avoiding the risk of unwanted hybridization by genetically low potential males during more dedicated breeding for weight gain, or for increasing the proportion of best meat quality compared to whole animals and accumulating fat, it becomes necessary in order to provide a better quality of the carcass.
As for the castration method, there are surgical methods such as partial castration, bloodless castration, etc. In addition, there is a method of irradiating a certain amount or more of radiation sensitive to the gonads to lose their ability.
However, physical castration is a problem in terms of animal welfare since it causes very serious pain and stress to animals. European countries such as Switzerland, Norway, Belgium, and the Netherlands decided to ban physical castration of animals in about 2009, and similar measures are being considered in Korea (Thun R et al., Castration in male pigs: Techniques and animal welfare issues. Journal of Physiology and Pharmacology, 57, pp.189-194, 2006).
Another method suitable for carrying out sterilization and libido removal is a chemical method. In the 1960s, research began to use a sclerosing material injected directly into the testis or spermatic cord for the purpose of promoting total loss of functions of the testis or spermatic cord (sperm and androgen hormone production).
Chemical sterilization has been attempted in monkeys, hamsters, rabbits, rats, and dogs by intratesticular administration of several agents as follows: ferrous chloride (Kar et al. 1965), Danazol (Dixit et al., 1975), BCG (Das et al. 1982), tannin zinc (Fahim et al., 1982), glycerol (Weinbauer et al., 1985, Immegart 2000), glucose, NaCl (Heath et al., 1987, Russell et al. 1987), DBCP (Shemi et al. 1988), lactic acid (Fordyce et al. 1989), zinc arginine (Fahim et al. 1993), sodium fluoride (Sprando et al. 1996), formalin (Balar et al. 2002), calcium chloride (Samanta 1998, Jana et al. 2002), and potassium permanganate + glacial acetic acid (Giri et al. 2002). In ruminants, a method of injecting lactic acid (Hill et al. 1985), tannic acid, zinc sulfate (Feher et al. 1985), alpha hydroxypropionic acid (Cohen et al. 1995), formalin (Ijaz et al. 2000), and CastrateQuin 14 (Soerensen et al. al. 2001) into the male testes was used.
Recently, deslorelin, a synthetic analogue of GnRH (gonadotropin-releasing hormone), was used for chemical castration of male animals and treatment of benign prostatic hyperplasia in dogs.
However, deslorelin was injected into the body of the animal as an implant insertion type, and there was a problem accompanying great pain to the animal when administered.
Accordingly, when deslorelin is used as a chemical castration agent, it is necessary to develop a product that can maintain the effect thereof as a temporary sterilant for a long time without causing significant pain to the animal.

[Prior Art Document]

[Patent Documents]

(Patent Document 0001) KR 10-2012-0052355 A1

### SUMMARY

An object of the present disclosure is to provide a composition for sustained-release injection comprising deslorelin.
Another object of the present disclosure is to provide a composition for sustained-release injection comprising deslorelin, the composition comprising microparticles which prevent initial over-release by adjusting the release rate of deslorelin at a target site, can exhibit the effect of deslorelin for 1 month or more by being exposed in an amount sufficient to show the effect of deslorelin, and are uniform.

Another object of the present disclosure is to provide a method for preparing a composition for sustained-release injection comprising deslorelin, the method which can prepare microparticles that have a uniform particle size and are capable of continuously exhibiting the effect of releasing deslorelin for a long time.

In order to achieve the above object, the present disclosure is a composition for sustained-release injection comprising deslorelin, the composition comprising microparticles, wherein the microparticles comprise deslorelin and a biodegradable polymer, and a value according to the following Equation 1 may be 1 to 10:

$$[\text{Equation 1}]$$

$$C_{max\ 1d\text{-}28d}/C_{max\ 0\text{-}1d}$$

where,

the composition for sustained-release injection comprising deslorelin is administered as an injection product to a beagle dog to measure the blood concentration of deslorelin,

$C_{max\ 1d\text{-}28d}$ is the maximum blood concentration of deslorelin within 1 to 28 days after injecting the injection product, and

$C_{max\ 0\text{-}1d}$ is the maximum blood concentration of deslorelin within 1 day after injecting the injection product.

The microparticles may have a value according to the following Equation 2 of 1 to 2:

$$[\text{Equation 2}]$$

$$\frac{D90 - D50}{D50 - D10}$$

where

D 10 is the diameter of the particles corresponding to 10% of the maximum value in the cumulative distribution of particles,

D50 is the diameter of the particles corresponding to 50% of the maximum value in the cumulative distribution of particles, and

D90 is the diameter of the particles corresponding to 90% of the maximum value in the cumulative distribution of particles.

The biodegradable polymer may be selected from the group consisting of polylactic acid, polylactide, polylactic-co-glycolic acid, polylactide-co-glycolide (PLGA), polyphosphazines, polyiminocarbonates, polyphosphoesters, polyanhydrides, polyorthoesters, polycaprolactone, polyhydroxyvalate, polyhydroxybutyrate, polyamino acids, and combinations thereof.

The biodegradable polymer may have a viscosity of 0.1 to 1.0 dl/g.

Deslorelin and the biodegradable polymer may be contained at a weight ratio of 1:4 to 1:10.

When administered as an injection product, the release rate of deslorelin at the target site may be adjusted, and the inhibitory effect of testosterone due to deslorelin may last for 1 month or more.

Testosterone may be maintained at 0.4 ng/ml or less after 10 days have passed since the injection product is administered.

The composition for injection may comprise a suspension solvent.

A method for preparing a composition for sustained-release injection comprising deslorelin according to another embodiment of the present disclosure may comprise the steps of: 1) preparing a first mixture by mixing deslorelin and a biodegradable polymer; 2) preparing a second mixture by dissolving a surfactant in a solvent; 3) injecting the first mixture and the second mixture into a first microchannel and a second microchannel respectively in which an intersection point is formed, and allowing them to flow therethrough to produce microparticles at the intersection point; 4) collecting the microparticles in a water tank containing the second mixture; 5) removing an organic solvent present in the collected microparticles; 6) washing the organic solvent-removed microparticles with purified water and drying the washed microparticles; and 7) mixing the dried microparticles with a suspension solution, and a value according to the following Equation 1 may be 1 to 10:

$$[\text{Equation 1}]$$

$$C_{\text{max 1d-28d}}/C_{\text{max 0-1d}}$$

where,

the composition for sustained-release injection comprising deslorelin is administered as an injection product to a beagle dog to measure the blood concentration of deslorelin,
$C_{\text{max 1d-28d}}$ is the maximum blood concentration of deslorelin within 1 to 28 days after injecting the injection product, and
$C_{\text{max 0-1d}}$ is the maximum blood concentration of deslorelin within 1 day after injecting the injection product.

When the first mixture is injected into the first microchannel, after the first mixture is injected under a pressure condition of 700 to 1,500 mbar, the pressure is increased to a first condition of 10 to 30 mbar/min, and when the injection pressure condition reaches 900 to 1,700 mbar, the pressure may be increased to a second condition of 2 to 8 mbar/min.

The second mixture may be injected into the second microchannel under a pressure condition that is 2 to 4 times greater than the pressure condition when the first mixture is injected into the first microchannel.

The step 5) may include the steps of: 5-1) performing primary stirring at a speed of 100 to 300 rpm for 20 to 40 minutes at 15 to 20°C; 5-2) performing secondary stirring at a speed of 100 to 300 rpm for 60 to 120 minutes at 30 to 40 °C; and 5-3) performing tertiary stirring at a speed of 100 to 300 rpm for 4 to 8 hours at 40 to 45°C.

The present disclosure, as a composition for sustained-release injection comprising deslorelin, comprises microparticles which can prevent initial over-release by adjusting the release rate of deslorelin at a target site, can exhibit the effect of deslorelin for 1 month or more by being exposed in an amount enough to show the effect of deslorelin, and are uniform.

Further, the microparticles can prepare microparticles that have a uniform particle size and are capable of exhibiting the effect of continuously releasing deslorelin for a long time.

**BRIEF DESCRIPTION OF THE DRAWINGS**

FIG. 1 is a result of measuring pK and PD values of Example 1 according to an embodiment of the present disclosure.

FIG. 2 is a result of measuring pK and PD values of Comparative Example 1 according to an embodiment of the present disclosure.

FIG. 3 is a result of comparing PD values of Example 1 and Comparative Example 1 according to an embodiment of the present disclosure.

FIG. 4 is a result of measuring pK and PD values of Example 2 according to an embodiment of the present disclosure.

FIG. 5 is a result of measuring pK and PD values of Comparative Example 2 according to an embodiment of the present disclosure.

FIG. 6 is a result of comparing PD values of Example 2 and Comparative Example 2 according to an embodiment of the present disclosure.

FIG. 7 is about the shaking direction for the elution experiment according to an embodiment of the present disclosure.

FIG. 8 is experimental results for the elution rates of Examples according to an embodiment of the present disclosure.

**DESCRIPTION OF SPECIFIC EMBODIMENTS**

Hereinafter, embodiments of the present disclosure will be described in detail so that those with ordinary skill in the art to which the present disclosure pertains can easily carry out the present disclosure. However, the present disclosure may be embodied in several different forms and is not limited to the embodiments described herein.

In the present disclosure, 'deslorelin' may comprise both L-pyroglutamyl-L-histidyl-L-tryptophyl-L-seryl-L-tyrosyl-D-tryptophyl-L-leucyl-L-arginyl-L-proline ethylamide and a pharmaceutically acceptable salt thereof.

In the present disclosure, 'pharmaceutically acceptable' means that it is physiologically acceptable and does not normally cause allergic reactions or reactions similar thereto when administered to humans.

In the present disclosure, the 'pharmaceutically acceptable salt' refers to an acid addition salt formed by a pharmaceutically acceptable free acid. An organic acid and an inorganic acid may be used as the free acid. The organic acid is not limited thereto, but includes citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, glutamic acid, and aspartic acid. Further, the inorganic acid is not limited thereto, but includes hydrochloric acid, bromic acid, sulfuric acid, and phosphoric acid.

The composition for sustained-release injection comprising deslorelin according to an embodiment of the present disclosure comprises microparticles, wherein the microparticles comprise deslorelin and a biodegradable polymer, and a value according to the following Equation 1 may be 1 to 10:

$$[\text{Equation 1}]$$

$$C_{max\ 1d\text{-}28d}/C_{max\ 0\text{-}1d}$$

where,

the composition for sustained-release injection comprising deslorelin is administered as an injection product to a beagle dog to measure the blood concentration of deslorelin,

$C_{max\ 1d\text{-}28d}$ is the maximum blood concentration of deslorelin within 1 to 28 days after injecting the injection product, and

$C_{max\ 0\text{-}1d}$ is the maximum blood concentration of deslorelin within 1 day after injecting the injection product.

Deslorelin is a gonadotropin-releasing hormone agonist (GnRH agonist).

Gonadotropin-releasing hormone (GnRH) is a natural hormone, which is produced by the hypothalamus, that interacts with receptors in the pituitary gland to stimulate the production of luteinizing hormone (LH).

In order to reduce LH production, agonists of the GnRH receptor (GnRH-R), such as leuprolide, goserelin, and the like, have been developed. These GnRH agonists are typically GnRH agonists and the decapeptide pyroGlu-His-Trp-SerTyr-Gly-Leu-Arg-Pro-Gly-NH2. For example, a GnRH agonist having the D-isomer instead of Gly in No. 6 has superior biological efficacy and higher binding affinity/strength to the receptor than the natural hormone.

The GnRH agonist may be used as a chemical castration agent, and may not be limited to the use, and uses that can be utilized by the effect of the GnRH agonist may all be used without limitation.

As described above, deslorelin is being used for chemical castration of animals. In order for the chemical castration effect by deslorelin to appear, the numerical value of testosterone should be maintained to 0.4 ng/ml or less. That is, deslorelin is a GnGH agonist, and may exhibit an inhibitory effect on testosterone.

However, in order to exhibit the effects as described above, it is known that when deslorelin is administered into the body by a method such as an injection product or the like, the effect can only be exhibited by exposing it to deslorelin to an excessive degree immediately after injection.

Specifically, GnRH agonists such as deslorelin initially act on the pituitary gland to promote gonadotropin secretion, but when continuously administered, the GnRH receptor is modified to inhibit gonadotropin secretion, and eventually the production of testosterone is also suppressed. Therefore, only after 1 to 3 weeks have passed, it is reduced to the level of castration by deslorelin (plasma testosterone <0.4ng/ml).

Due to the characteristics as described above, it has been recognized that an over-release of the drug at the initial stage of injection is necessary in order to rapidly exhibit the castration effect by deslorelin.

On the other hand, the composition for sustained-release injection according to the present disclosure is one that can exhibit the chemical castration effect for 1 month, 3 months, 6 months, 12 months, or 24 months. Unlike previously known contents, it may exhibit an excellent castration effect in the early stage of injection even when preventing excessive release of deslorelin.

Equation 1 uses the result of measuring the deslorelin blood concentration (pK) of the beagle dog after administering the composition for sustained-release injection comprising deslorelin according to the present disclosure.

A value according to Equation 1 may be 1 to 10, 3 to 10, 6 to 10, 7 to 10, 7.5 to 9.5, and 7.5 to 9. Within the above range, the initial over-release may be prevented, and the chemical castration effect by deslorelin continuously for a desired period may be achieved.

Specifically, Equation 1 is as follows:

[Equation 1]

$$C_{max\ 1d-28d}/C_{max\ 0-1d}$$

where

the composition for sustained-release injection comprising deslorelin is administered as an injection product to a beagle dog to measure the blood concentration of deslorelin,

$C_{max\ 1d-28d}$ is the maximum blood concentration of deslorelin within 1 to 28 days after injecting the injection product, and

$C_{max\ 0-1d}$ is the maximum blood concentration of deslorelin within 1 day after injecting the injection product.

$C_{max\ 1d-28d}$ is the maximum blood concentration of deslorelin within 1 to 28 days after injecting the injection product according to the present disclosure, and $C_{max\ 0-1d}$ is the maximum blood concentration of deslorelin within 1 day after injecting the injection product.

When the value according to Equation 1 above represents a value greater than 1, it means that after administering the composition for injection according to the present disclosure to a beagle dog, the blood concentration of deslorelin shows a higher numerical value after 1 day.

The initial over-release refers to the maximum blood concentration within 24 hours after the drug is administered into the body through an injection product or the like.

Since a general injection product administers a drug itself, the maximum blood concentration is displayed immediately after injection.

Further, in the case of a composition for sustained-release injection comprising microparticles, the drug may be attached to the surface of the microparticles in the preparation process, the size of the particles may not be uniform, the surface of the particles may be uneven, or the maximum blood concentration may be shown immediately after injection due to the difference by the combination of the drug and the biodegradable polymer.

Meanwhile, the composition for sustained-release injection according to the present disclosure is characterized in that it comprises microparticles comprising deslorelin, but does not show the maximum blood concentration within 1 day after injection so that the initial over-release is suppressed.

Specifically, modification of GnRH receptor occurred in the past so that the secretion of gonadotropin was suppressed, and in order to eventually suppress the production of testosterone, deslorelin was overexposed in the beginning.

However, according to the results of confirming the inhibitory effect of testosterone using currently commercially available products as described later, it was confirmed that the excellent inhibitory effect of testosterone did not appear even if there was an overexposure of deslorelin at the initial stage of injection.

This will mean that it is not necessary to excessively release deslorelin in the beginning in order to inhibit testosterone as previously known.

The present disclosure is a composition for sustained-release injection comprising microparticles capable of inhibiting initial over-release and exhibiting a maximum blood concentration within 4 weeks, and may exhibit a sustained testosterone inhibitory effect by allowing deslorelin to be released continuously for a desired period while suppressing the excessive release of deslorelin in the beginning.

When the range value according to Equation 1 above is satisfied, it means that a continuous testosterone inhibitory effect may be exhibited while preventing the initial over-release of deslorelin. A value according to Equation 1 above greater than 1 means that the maximum blood concentration of deslorelin within 1 to 28 days exhibits a value greater than the maximum blood concentration of deslorelin within 1 day.

According to the experimental results as described above, an over-release of deslorelin at the initial stage of injection is unnecessary in order to effectively suppress testosterone. Further, it should be exposed to testosterone in an amount of a certain level or more for 1 to 28 days after injection in order to exhibit a sustained testosterone inhibitory effect for a long time. This is because the inhibitory effect of testosterone appears due to the modification of the GnRH receptor. Continuous release of deslorelin is required for a certain period of time in order to modify and maintain the GnRH receptor.

As a result of measuring an AUC value within 1 week after injection into the beagle dog, the composition for sustained-release injection according to the present disclosure may have a measured AUC value of 500 to 1,500 day*pg/ml, 700 to 1,300 day*pg/ml, or 750 to 1,100 day*pg/ml. This is a level of about 12.9% compared to the conventional product, and it can be confirmed more clearly that there is no initial over-release.

Accordingly, in the present disclosure, it is characterized in that the value according to Equation 1 above is 1 to 10, and within the above range, an inhibitory effect of testosterone may be exhibited for 1 month, 3 months, 6 months, 12 months, or 24 months.

The microparticles may have a value according to the following Equation 2 of 1 to 2:
[Equation 2]

$$[\text{Equation 2}]$$
$$\frac{D90 - D50}{D50 - D10}$$

where

    D 10 is the diameter of the particles corresponding to 10% of the maximum value in the cumulative distribution of particles,

    D50 is the diameter of the particles corresponding to 50% of the maximum value in the cumulative distribution of particles,

    D90 is the diameter of the particles corresponding to 90% of the maximum value in the cumulative distribution of particles.

D10, D50, and D90 mean values corresponding to 10%, 50%, and 90% of the maximum values in the cumulative distribution for the measured diameters after measuring diameters of the microparticles.

Equation 2 above limits the ratio of (D90-D50) and (D50-D10), and is one which checks the degree of uniform particle distribution by checking the ratio of a difference between the diameter of the particles corresponding to 90% of the maximum value in the cumulative distribution of particles within the average particle distribution and the diameter of the particles corresponding to 50% thereof and a difference between the diameter of the particles corresponding to 50% thereof and the diameter of the particles corresponding to 10% thereof. It means that the closer the value is to 1, the more uniform the distribution width is.

Equation 2 of the present disclosure is to more clearly confirm the size distribution of the microparticles, and the value according to Equation 2 may be 1 to 2, 1.1 to 1.9, 1.2 to 1.8, or 1.3 to 1.7. When the value according to Equation 2 above is satisfied and the average diameter of the microparticles is 65 to 100 μm at the same time, it means that the size of the microparticles is distributed close to the average diameter value. In this case, microparticles with a uniform size are injected into the body through injection, the microparticles with a uniform size are biodegraded to a similar degree, and the release effect of deslorelin may be exhibited by biodegradation of the microparticles.

That is, in the case of microparticles comprising deslorelin, the degree of release of deslorelin in the body is highly correlated with the size and specific surface area of the particles, and in order to increase the specific surface area, it is essential to use microparticles having a uniform diameter. Initial over-release during injection into the body may be prevented by using microparticles having a very uniform particle size as described above, and the effect of releasing deslorelin continuously for a long time may be exhibited so that the castration effect by deslorelin may be exhibited for 1 month or more.

The weight ratio of deslorelin and the biodegradable polymer may be 1:4 to 1:10, 1:5 to 1:8, 1:6 to 1:7, or 1:6.6667. When they are used by mixing them within the above range, deslorelin may be continuously released for a long time by decomposition of the biodegradable polymer.

The biodegradable polymer may be selected from the group consisting of polylactic acid, polylactide, polylactic-co-glycolic acid, polylactide-co-glycolide (PLGA), polyphosphazines, polyiminocarbonates, polyphosphoesters, polyanhydrides, polyorthoesters, polycaprolactone, polyhydroxyvalate, polyhydroxybutyrate, polyamino acids, and combinations thereof, and may be preferably polylactide-co-glycolide (PLGA) or polylactide (PLA), but is not limited to the above examples.

The biodegradable polymer may have a viscosity of 0.1 to 1.0 dl/g. As described above, the sustained release of deslorelin is also affected by the average diameter, size distribution, and surface state of the microparticles, but there may also be a large difference due to the combination of the biodegradable polymer and drug in addition to the above factors.

The biodegradable polymer is a product that can be injected into the body, and may have a viscosity range of 0.1 to 1.0 dl/g, 0.1 to 0.9 dl/g, 0.15 to 0.8 dl/g, 0.15 to 0.7 dl/g, or 0.2 to 0.65 dl/g. When the biodegradable polymer is used within the above range, the initial over-release is inhibited by the combination with deslorelin, but an appropriate degree of release of deslorelin appears even at the beginning of injection, and the effect of releasing deslorelin continuously may be exhibited for 1 month, 3 months, 6 months, 12 months, or 24 months.

Further, the microparticles according to the present disclosure may be prepared using one biodegradable polymer or two or more biodegradable polymers.

Specifically, the microparticles contained in the composition for sustained-release injection according to the present disclosure may contain only one biodegradable polymer or may contain two or more biodegradable polymers. A plurality of the microparticles may be contained in the composition for sustained-release injection, and the microparticles may individually contain a separate biodegradable polymer.

That is, the microparticles comprising one biodegradable polymer may be contained in the composition for injection, and the microparticles comprising different biodegradable polymers may be contained in the composition for injection.

The biodegradable polymer may include a polymer showing a viscosity range of 0.1 to 0.3 dl/g or 0.3 to 0.7 dl/g.

Specifically, microparticles selected from the group consisting of microparticles comprising a biodegradable polymer having a viscosity of 0.1 to 0.3 dl/g, microparticles comprising a biodegradable polymer having a viscosity of 0.3 to 0.7 dl/g, and a mixture thereof may be contained in a composition for sustained-release injection.

The biodegradable polymer having a viscosity of 0.1 to 0.3 dl/g may have a viscosity of specifically 0.13 to 0.28 dl/g, 0.15 to 0.25 dl/g, or 0.22 dl/g. The composition for sustained-release injection may prevent the initial over-release of deslorelin and exhibit the effect of continuously releasing deslorelin for 1 month, 3 months, 6 months, 12 months, or 24 months by comprising the biodegradable polymer exhibiting the above viscosity range.

The biodegradable polymer having a viscosity of 0.3 to 0.7 dl/g may have a viscosity of specifically 0.45 to 0.65 dl/g, 0.5 to 0.6 dl/g, or 0.57 dl/g. The composition for sustained-release injection may prevent the initial over-release of deslorelin and exhibit the effect of continuously releasing deslorelin for 1 month, 3 months, 6 months, 12 months, or 24 months by comprising the biodegradable polymer exhibiting the above viscosity range.

Further, the microparticles comprising the biodegradable polymer having a low viscosity are decomposed relatively quickly in the body so that the release of deslorelin may be terminated, and the microparticles comprising the biodegradable polymer having a high viscosity are decomposed relatively slowly in the body so that the initial release of deslorelin may not be shown.

Accordingly, microparticles comprising two or more biodegradable polymers having different viscosities are prepared respectively, and all of these microparticles are contained in the composition for sustained-release injection so that the release of deslorelin may be shown even in the beginning, and the effect of continuously releasing deslorelin for a long time may be exhibited.

As will be described later, the microparticles comprising different biodegradable polymers may be contained in the composition for injection by the preparation method according to the present disclosure. That is, in the conventional method for preparing microparticles comprising biodegradable polymers, since the size of particles could not be controlled so that it has been impossible to prepare particles with a uniform size, it has been impossible to provide a composition for sustained-release injection by preparing microparticles using different biodegradable polymers and mixing the prepared microparticles.

However, as described later in the present disclosure, a preparation method capable of controlling the size of the particles and preparing particles having very uniform particle size and even surface shape is used so that the particles may be prepared into microparticles each comprising deslorelin using two or more biodegradable polymers, and a composition for sustained-release injection may be provided by mixing the prepared microparticles.

A method for preparing a composition for sustained-release injection comprising deslorelin according to another embodiment of the present disclosure may comprise the steps of: 1) preparing a first mixture by mixing deslorelin and a biodegradable polymer; 2) preparing a second mixture by dissolving a surfactant in a solvent; 3) injecting the first mixture and the second mixture into a first microchannel and a second microchannel respectively in which an intersection point is formed, and allowing them to flow therethrough to produce microparticles at the intersection point; 4) collecting the microparticles in a water tank containing the second mixture; 5) removing an organic solvent present in the collected microparticles; 6) washing the organic solvent-removed microparticles with purified water and drying the washed microparticles; and 7) mixing the dried microparticles with a suspension solution.

The step 1), as a step of preparing a first mixture, is a step of dissolving deslorelin and a biodegradable polymer in an organic solvent to prepare the first mixture, wherein the biodegradable polymer may be selected from the group consisting of polylactic acid, polylactide, polylactic-co-glycolic acid, polylactide-co-glycolide (PLGA), polyphosphazines, polyiminocarbonates, polyphosphoesters, polyanhydrides, polyorthoesters, polycaprolactone, polyhydroxyvalate, polyhydroxybutyrate, polyamino acids, and combinations thereof, and may be preferably polylactide-co-glycolide (PLGA) or polylactide (PLA), but is not limited to the above examples.

Further, the organic solvent may be one which is not mixed with water, and may be, for example, one or more selected from the group consisting of chloroform, chloroethane, dichloroethane, trichloroethane, and mixtures thereof, preferably dichloromethane, but is not limited to the examples. The organic solvent, as an organic solvent capable of dissolving biodegradable polymers and deslorelin, is not limited to the above examples, and any organic solvent that can be easily selected by those skilled in the art will be usable.

The step 1) is to prepare a first mixture in which deslorelin and a biodegradable polymer are dissolved, and an organic solvent as a solvent is used as described above. They are completely dissolved by using the dissolution properties of deslorelin and the biodegradable polymer, thereby using the organic solvent.

More specifically, deslorelin acetate is dissolved in a first solvent, and the biodegradable polymer is dissolved in a second solvent. Thereafter, a deslorelin acetate mixture dissolved in the first solvent and the biodegradable polymer mixture dissolved in the second solvent are mixed to prepare a first mixture.

The first mixture may have a weight ratio of deslorelin and the biodegradable polymer of 1:4 to 1:10, 1:5 to 1:8, 1:6 to 1:7, or 1:6.6667. When they are used by mixing them within the above range, deslorelin may be continuously released for a long time by decomposition of the biodegradable polymer.

When the weight ratio of deslorelin and the biodegradable polymer is less than 1:4, that is, when the biodegradable polymer is contained in an amount less than the weight ratio, the ratio of the weight of the biodegradable polymer to that of deslorelin is small so that there may be a problem in that it is difficult to prepare sustained-release particles in the form in which deslorelin is uniformly distributed and contained in spherical biodegradable polymer particles. When the weight ratio of deslorelin and the biodegradable polymer is more than 1:10, that is, when the biodegradable polymer is contained in an amount more than the weight ratio, the amount of deslorelin contained in sustained-release particles is small so that there may be a problem in that a large amount of the sustained-release particles should be administered in order to administer the drug at a desired concentration.

More specifically, the biodegradable polymer may be contained in the first mixture in an amount of 15 to 25% by weight, preferably 20% by weight, but is not limited to the above examples.

The step 2), as a step of preparing a second mixture, dissolves a surfactant in water to prepare the second mixture. The surfactant can be used without limitation as long as it can help form an emulsion in which a biodegradable polymer solution is stable. Specifically, the surfactant is one or more selected from the group consisting of nonionic surfactants, anionic surfactants, cationic surfactants, and mixtures thereof, more specifically, one or more selected from the group consisting of methylcellulose, polyvinylpyrrolidone, lecithin, gelatin, polyvinyl alcohol, polyoxyethylene sorbitan fatty acid esters, polyoxyethylene castor oil derivatives, sodium lauryl sulfate, sodium stearate, ester amines, linear diamines, fatty amines, and mixtures thereof, and preferably polyvinyl alcohol, but is not limited to the examples.

The surfactant contained in the second mixture may be contained in an amount of 0.1 to 1.0% by weight, 0.3 to 0.7% by weight, or 0.5% by weight. The residual amount of water may be contained therein.

The step 3) is a step of injecting the first mixture and the second mixture into the microchannel formed on the wafer to allow them to flow therethrough.

More specifically, the microchannel may be formed on a material selected from the group consisting of a silicon wafer and a polymer film, but examples of the material are not limited to the above examples, and any material capable of forming a microchannel may be usable.

The polymer film may be selected from the group consisting of polyimide, polyethylene, fluorinated ethylene propylene, polypropylene, polyethylene terephthalate, polyethylene naphthalate, polysulfone, and mixtures thereof, but is not limited to the above examples.

As an example, aluminum is deposited on a silicon wafer using an e-beam evaporator, and a photoresist is patterned on aluminum using a photolithography technique. Thereafter, aluminum etching is performed using the photoresist as a mask, and after the photoresist is removed, silicon is etched by deep ion reactive etching (DRIE) using aluminum as a mask, and after aluminum is removed, the microchannel is prepared by anodic bonding and sealing glass on the wafer.

The microchannel has an average diameter of 80 to 120 $\mu$m, preferably 100 $\mu$m, but is not limited to the examples. Since, when the average diameter of the microchannel is 80 $\mu$m or less, sustained-release particles prepared have a diameter of less than 40 $\mu$m so that there is a possibility that small sustained-release particles may be prepared, the release of effective drugs and absorption thereof in vivo may be affected. Further, when the prepared sustained-release particles have an average size of more than 100 µm, foreign body sensation and pain may increase when administered as an injection product, and the particle size distribution of the prepared particles increases so that it may be difficult to prepare sustained-release particles with a uniform particle size.

However, the average diameter of the microchannel may be changed according to the range of the injection pressure. Further, the average diameter of the microchannel is closely related to the average diameter of the particles, but is also closely related to the injection pressure of the first mixture and the second mixture.

Further, the cross-sectional width (w) and the cross-sectional height (d) of the microchannel are closely related to the average diameter (d') of the sustained-release particles prepared. The cross-sectional width (w) of the microchannel is in a ratio range of 0.7 to 1.3 with respect to the average diameter (d') of the sustained-release particles, and the cross-sectional height (d) of the microchannel is in a ratio range of 0.7 to 1.3 with respect to the average diameter (d') of the sustained-release particles.

That is, if the average diameter (d') of the sustained-release particles to be prepared is determined, the length of the cross-sectional width (w) and the cross-sectional height (d) of the microchannel should be set in a ratio range of 0.7 to 1.3 of d' accordingly so that sustained-release particles with a desired size can be prepared.

The step 3) is allowing the first mixture and the second mixture to flow into the first microchannel and the second microchannel in which an intersection point is formed, under the injection pressure condition.

That is, the first mixture flows along the first microchannel, and the second mixture flows along the second microchannel which is formed to form an intersection point with the first microchannel, and meets the flow of the first mixture.

More specifically, when the first mixture is injected into the first microchannel, after it is injected under a pressure condition of 700 to 1,500 mbar, the pressure is increased to a first condition of 10 to 30 mbar/min, and when the injection pressure condition reaches 900 to 1,700 mbar, the pressure may be increased to a second condition of 2 to 8 mbar/min.

Further, the second mixture may be injected into the second microchannel under a pressure condition that is 2 to 4 times greater than a pressure condition when the first mixture is injected into the first microchannel.

Specifically, in a preparation method using the microchannel, when the flow rates of the first mixture and the second mixture flowing through the microchannel are set to constant values using a flowmeter and the pressure is measured through feedback control, it is confirmed that the pressure required to flow the first mixture through the microchannel at a constant flow rate is gradually increased with time.

Therefore, a method of constantly increasing the pressure applied to the first mixture may be used to minimize the flow rate variability, prevent the problem of microparticle distribution nonuniformity or channel closure due to slow curing of the first mixture inside the microchannel, and increase the target preparation yield of the microparticles.

Further, the pressure condition when the first mixture and the second mixture are injected into the microchannel is for adjusting the average diameter of the prepared microparticles, and when the above range is not specifically satisfied, there may be a problem in that the size of the prepared particles is not uniform, the average diameter range of the microparticles according to the present disclosure is not satisfied, or the value according to Equation 1 above is not satisfied.

That is, in order to allow the flow of the second mixture forming an intersection point with the flow of the first mixture to flow at a faster flow rate than the flow of the first mixture injected into a microchannel of the straight direction, the second mixture is allowed to flow under a higher pressure condition.

As described above, the flow rates of the first mixture and the second mixture are differentiated, and the flow rate of the second mixture is made faster than the flow rate of the first mixture so that the second mixture having a relatively faster flow rate compresses the first mixture at a point where the flow of the first mixture and the flow of the second mixture meet. At this time, the biodegradable polymer and deslorelin in the first mixture produce spherical microparticles due to the repulsive force of the first mixture and the second mixture, and more specifically, microparticles are formed in a form in which deslorelin is evenly distributed in a spherical biodegradable polymer.

In the step 4), as a step of collecting the microparticles, the microparticles are collected in a water tank containing the second mixture to prevent an aggregation phenomenon from occurring between initially produced microparticles.

The step 4) is one which uses the second mixture prepared in the step 2), that is, a mixed solution of the surfactant and water. After the second mixture is prepared in the step 2), a portion is injected into the microchannel, and another portion is moved to the water tank of the step 4) and used to prevent the aggregation phenomenon from occurring between the collected microparticles.

In the step 5), as a step for removing the organic solvent present in the microparticles collected in the water tank, the organic solvent present on the surface of the sustained-release particles is evaporated and removed by performing stirring under stirring conditions such as constant temperature condition and stirring speed. At this time, the stirring conditions include: a step 5-1) of performing primary stirring at a speed of 100 to 300 rpm for 20 to 40 minutes at 15 to 20 °C; a step 5-2) of performing secondary stirring at a speed of 100 to 300 rpm for 60 to 120 minutes at 30 to 40 °C; and a step 5-3) of performing tertiary stirring at a speed of 100 to 300 rpm for 4 to 8 hours at 40 to 45°C.

The stirring step is performed by varying the temperature condition and the stirring progress time in the primary and secondary stirring steps to proceed with the stirring process.

As described above, it is characterized in that the temperature condition is increased in the secondary stirring process compared to the primary stirring process to perform stirring, and as the temperature is raised stepwise, the evaporation rate of the organic solvent present on the surface of the microparticles may be adjusted. That is, microparticles may be prepared by slowly evaporating the organic solvent present on the surface of the microparticles.

The temperature at which the first mixture and the second mixture flow through the microchannel is also 15 to 20°C, preferably 17°C. That is, after flowing them through the microchannel and forming an intersection point to produce microparticles, a low temperature is constantly maintained at 15 to 20°C until the collected microparticles are subjected to primary stirring. It is possible to prepare and maintain spherical particles only by maintaining a low temperature in the preparation process of microparticles. That is, when it is not a low temperature condition, there may be a problem in that it is difficult to prepare particles having a constant spherical shape.

Thereafter, the effect of the organic solvent on the surface of the microparticles may be minimized as the organic solvent evaporates from the surface by gradually raising the temperature and increasing the stirring time in the secondary stirring process and the tertiary stirring process, thereby slowly evaporating the organic solvent present on the surface of the microparticles. That is, when the organic solvent is rapidly evaporated, there may be a problem in that the surface of the microparticles is not smooth and becomes rough due to the evaporation of the organic solvent. In order to prevent such a problem, as described above, the temperature condition may be gradually increased, and the time for proceeding the stirring process may also be increased to adjust the evaporation rate of the organic solvent, and the surface roughness of the prepared microparticles may be controlled due to adjustment of the evaporation rate of such an organic solvent.

Finally, in the step 6), which is as a step of washing and drying the microparticles, the microparticles from which the organic solvent on the surface is all removed by stirring are washed several times with sterilized filtered purified water to remove the surfactant remaining in the microparticles, and then freeze-dried.

The microparticles finally produced are in a form in which deslorelin is evenly distributed in microparticles comprised of a spherical biodegradable polymer, and comprise deslorelin and the biodegradable polymer at a weight ratio of 1:4 to 1:10.

The weight ratio of deslorelin and the biodegradable polymer contained in the microparticles is the same as the weight ratio in the first mixture, which enables microparticles containing deslorelin and the biodegradable polymer at the same ratio as the weight ratio in the first mixture to be prepared by preparing the microparticles and evaporating and removing all of the organic solvent.

The prepared microparticles may be mixed with a suspension solvent to prepare a composition for injection.

The suspension solvent includes an isotonic agent, a suspending agent, and a solvent.

More specifically, the isotonic agent may be selected from the group consisting of D-mannitol, maltitol, sorbitol, lactitol, xylitol, sodium chloride, and mixtures thereof, preferably D-mannitol, but is not limited to the above examples.

The suspending agent may be selected from the group consisting of sodium carboxymethyl cellulose, polysorbate 80, starch, starch derivatives, polyalcohols, chitosan, chitosan derivatives, cellulose, cellulose derivatives, collagen, gelatin, hyaluronic acid (HA), alginic acid, algin, pectin, carrageenan, chondroitin, chondroitin sulfate, dextran, dextran sulfate, polylysine, titin, fibrin, agarose, fluran, xanthan gum, and mixtures thereof, preferably sodium carboxymethyl cellulose and polysorbate 80, but is not limited to the above examples.

Water for injection may be used as the solvent, and any solvent that can be used as water for injection may be used without limitation.

Preparation Example 1

Preparation of Microparticles Comprising Deslorelin

An API mixture was prepared by dissolving deslorelin acetate in methanol. A biodegradable polymer mixture was prepared by dissolving PLGA and PLA having a viscosity of 0.22 dl/g in dichloromethane. The weight ratio of PLGA and PLA in the biodegradable polymer mixture is 1.7 to 18.3.

A first mixture was prepared by mixing the API mixture and the biodegradable polymer mixture. At this time, the weight ratio of deslorelin acetate and a biodegradable polymer in the first mixture is 1:6.6667.

A second mixture comprising 0.5% by weight of polyvinyl alcohol was prepared by mixing polyvinyl alcohol, which is a surfactant, with water.

The first mixture and the second mixture were injected into microchannels formed on a silicon wafer to flow.

At this time, in order to flow the first mixture and the second mixture at a constant flow rate, after starting with a pressure condition of 1,000 mbar and allowing the first mixture to flow under a condition of constantly increasing the pressure at an increase rate of 20 mbar per minute, the first mixture was allowed to flow under the conditions that had been changed to an increase rate of 7 mbar per minute when it reached 1,200 mbar, and the second mixture was allowed to flow under the pressure condition of 3,000 mbar. The temperature condition was maintained at 17E, and the stirring speed was maintained at 300 rpm.

The microparticles produced at the intersection point where the flow of the first mixture and the flow of the second mixture meet were collected in a water tank containing the second mixture. The microparticles collected in the water tank were subjected to primary stirring at a speed of 300 rpm for 30 minutes at 17É, the temperature was raised to 38É to perform secondary stirring at a speed of 400 rpm for 1 hour, and then the temperature was raised to 45É to perform tertiary stirring at a speed of 500 rpm for 3 hours.

The stirring-completed microparticles were washed several times with sterilized filtered purified water and freeze-dried to prepare microparticles.

Preparation Example 2

PLA having a viscosity of 0.37 dl/g as a biodegradable polymer was dissolved in methanol to prepare a biodegradable polymer mixture, and microparticles were prepared in the same manner as in Preparation Example 1.

Preparation Example 3

Microparticles were prepared in the same manner as in Preparation Example 1 except that PLA having a viscosity of 0.57 dl/g as a biodegradable polymer was dissolved in methanol to prepare a biodegradable polymer mixture.

Preparation Example 4

Microparticles were prepared in the same manner as in Preparation Example 1 except that PLGA having a viscosity of 0.22 dl/g and PLA having a viscosity of 0.41 dl/g were dissolved in methanol to prepare a biodegradable polymer mixture, and the weight ratio of PLGA and PLA in the biodegradable polymer mixture was 1.7 to 18.3.

Example 1

The microparticles of Preparation Example 1 were added to 2.0 ml of a suspension solvent based on 1 vial, and then uniformly suspended to prepare a composition for subcutaneous injection. The weight ratio of deslorelin acetate and a biodegradable polymer in the composition for subcutaneous injection is 1:6.6667.

The suspension solvent was composed of the composition as shown in Table 2 below.

[Table 1]

| Content standard | Purpose of mixing | Ingredient name | Amount | Unit |
|---|---|---|---|---|
| 2.0 mL | Isotonic agent | D-Mannitol | 100.0 | mg |
| | Suspending agent | Sodium carboxymethylcellulose | 5.0 | mg |
| | Suspending agent | Polysorbate 80 | 2.0 | mg |
| | Solvent | Water for injection | The remaining amount | |

Example 2

A composition for subcutaneous injection was prepared in the same manner as in Example 1 except that the microparticles of Preparation Example 1 and the microparticles of Preparation Example 2 were mixed at a weight ratio of 1:0.92 and used.

Example 3

A composition for subcutaneous injection was prepared in the same manner as in Example 1 except that the microparticles of Preparation Example 1 and the microparticles of Preparation Example 3 were mixed at a weight ratio of 1:0.92 and used.

Example 4

A composition for subcutaneous injection was prepared in the same manner as in Example 1 except that the microparticles of Preparation Example 4 were mixed and used instead of the microparticles of Preparation Example 1.

Comparative Example 1

As a comparative example, a commercially available Suprelorin 4.7 mg Implant (containing 4.7 mg of deslorelin) was used.

Comparative Example 2

As a comparative example, a commercially available Suprelorin 9.4 mg Implant (containing 9.4 mg of deslorelin) was used.

Experimental Example 1

Examination of the properties of microparticles

In order to specifically confirm the diameter of the microparticles, the analysis was performed using a Microtrac particle size analyzer.

[Table 2]

| %Tile | Preparation Example 1 | Preparation Example 2 | Preparation Example 3 | Preparation Example 4 |
|---|---|---|---|---|
| 10.00 | 69.65 | 88.23 | 63.14 | 74.21 |
| 20.00 | 73.49 | 90.24 | 64.74 | 76.84 |
| 30.00 | 75.91 | 92.18 | 66.06 | 79.08 |
| 40.00 | 78.07 | 93.82 | 67.33 | 81.24 |
| 50.00 | 80.09 | 95.45 | 68.62 | 83.38 |
| 60.00 | 82.19 | 97.15 | 69.92 | 85.75 |
| 70.00 | 84.34 | 98.87 | 71.33 | 88.41 |
| 80.00 | 87.07 | 100.7 | 73.01 | 92.44 |
| 90.00 | 91.63 | 103.1 | 75.76 | 98.69 |
| 95.00 | 96.43 | 104.3 | 78.49 | 103.7 |
| **D90-D50 D50-D10** | 1.11 | 1.06 | 1.30 | 1.67 |

(Unit $\mu$m) As shown in Table 2 above, the average diameters (D50) as results of analyzing particle sizes for Preparation Examples were confirmed to be 80.09 $\mu$m, 95.45 $\mu$m, 68.62 $\mu$m, and 83.38 $\mu$m. It was confirmed that the values according to Equation in Table 2 above were also 1.11, 1.06, 1.30, and 1.67, which were all included in the range of 1 to 2, so that they were included as particles having a very uniform diameter from D10 to D95.

Experimental Example 2

Evaluation 1 of Pharmacokinetic Properties

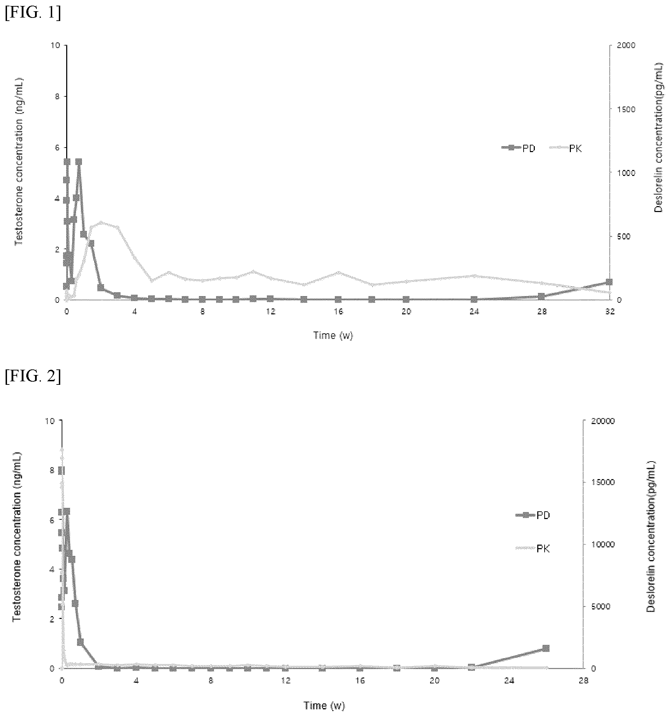

Pharmacokinetic evaluation for Example 1 and Comparative Example 1 of the present disclosure was confirmed.
For evaluation, Example 1 and Comparative Example 1 were administered to a beagle dog, and blood was collected to measure the blood concentration of deslorelin (PK) and the blood concentration of testosterone (PD).
The injection product comprising the microparticles of Preparation Example 1 used as a sustained-release formulation for 6 months or more contained 6.11 mg of deslorelin.
For the experiment, Example 1 and Comparative Example 1 were administered to each of 5 beagle dogs, and a subcutaneous injection method was used as the route of administration.
After blood collection, the mean values of PK and PD for 5 beagle dogs were calculated. The experimental results are as shown in Table 3, Table 4, and FIGS. 1 to 3 below.

[Table 3]

| PK | Time (w) | Time (d) | Time (h) | pk (pg/ml) | PD (ng/ml) |
|---|---|---|---|---|---|
| | 0.000 | 0 | 0 | 0 | 0.510 |
| | 0.001 | 0.01 | 0.25 | 19 | 1.417 |
| | 0.003 | 0.02 | 0.5 | 53 | 1.718 |
| | 0.006 | 0.04 | 1 | **68** | 3.896 |
| | 0.012 | 0.08 | 2 | 24 | 4.680 |
| | 0.018 | 0.13 | 3 | 31 | 5.399 |
| | 0.036 | 0.25 | 6 | 9 | 3.058 |
| | 0.071 | 0.5 | 12 | 18 | 1.634 |
| | **0.143** | **1** | **24** | 29 | 1.734 |
| | 0.286 | 2 | 48 | 25 | 0.720 |
| | 0.429 | 3 | 72 | 34 | 3.127 |
| | 0.571 | 4 | 96 | 162 | 3.965 |
| | 0.714 | 5 | 120 | 196 | 5.390 |
| | 1 | 7 | 168 | 305 | 2.547 |
| | 1.4 | 10 | 240 | 569 | 2.214 |
| | 2 | 14 | 336 | **605** | 0.443 |
| | 3 | 21 | 504 | 570 | 0.155 |
| | **4** | **28** | **672** | 327 | 0.078 |
| | 5 | 35 | 840 | 148 | 0.024 |
| | 6 | 42 | 1008 | 210 | 0.020 |
| **Example 1** | 7 | 49 | 1176 | 159 | 0.005 |
| | 8 | 56 | 1344 | 147 | 0.005 |
| | 9 | 63 | 1512 | 169 | 0.014 |
| | 10 | 70 | 1680 | 173 | 0.012 |
| | 11 | 77 | 1848 | 222 | 0.019 |
| | 12 | 84 | 2016 | 171 | 0.021 |
| | 14 | 98 | 2352 | 116 | 0.005 |
| | 16 | 112 | 2688 | 216 | 0.000 |
| | 18 | 126 | 3024 | 114 | 0.000 |
| | 20 | 140 | 3360 | 143 | 0.005 |
| | 24 | 168 | 4032 | 189 | 0.010 |
| | 28 | 196 | 4704 | 127 | 0.138 |
| | 32 | 224 | 5376 | 58 | 0.691 |
| Equation 1 <br> Cmax $_{1d-28d}$/Cmax $_{0-1d}$ | | | | 8.9 | - |

Table 3 above is for pK and PD values for Example 1, and relates to a sustained-release injection product composition capable of continuously releasing deslorelin for 6 months. In order to derive the value according to Equation 1 above, the maximum blood concentration of deslorelin for 1 to 28 days is 605 pg/ml, and the maximum blood concentration of deslorelin within 1 day is 68 pg/ml. It can be confirmed that the value according to Equation 1 is 8.9, which is included within the range of the present disclosure.

Further, it was confirmed that the time point at which the testosterone inhibitory effect appeared as a PD value of 0.4 ng/ml or less started to appear from the time point when 2 weeks had elapsed.

[Table 4]

| | Time (w) | Time (d) | Time (h) | pk (pg/ml) | PD (ng/ml) |
|---|---|---|---|---|---|
| Comparative Example 1 | 0.000 | 0 | 0 | 0 | 2.456 |
| | 0.001 | 0.01 | 0.25 | 7681 | 2.856 |
| | 0.003 | 0.02 | 0.5 | 14959 | 5.447 |
| | 0.006 | 0.04 | 1 | **17624** | 6.278 |
| | 0.012 | 0.08 | 2 | 16941 | 7.925 |
| | 0.018 | 0.13 | 3 | 14588 | 7.997 |
| | 0.036 | 0.25 | 6 | 5115 | 4.823 |
| | 0.071 | 0.5 | 12 | 1452 | 3.614 |
| | **0.143** | **1** | **24** | **697** | 3.132 |
| | 0.286 | 2 | 48 | 285 | 6.336 |
| | 0.429 | 3 | 72 | 325 | 4.630 |
| | 0.571 | 4 | 96 | 318 | 4.364 |
| | 0.714 | 5 | 120 | 355 | 2.588 |
| | 1 | 7 | 168 | 360 | 1.041 |
| | 2 | 14 | 336 | 353 | 0.060 |
| | 3 | 21 | 504 | 291 | 0.011 |
| | **4** | **28** | **672** | 313 | 0.016 |
| | 5 | 35 | 840 | 276 | 0.005 |
| | 6 | 42 | 1008 | 298 | 0.000 |
| | 7 | 49 | 1176 | 213 | 0.000 |
| | 8 | 56 | 1344 | 208 | 0.005 |
| | 9 | 63 | 1512 | 178 | 0.000 |
| | 10 | 70 | 1680 | 242 | 0.005 |
| | 11 | 77 | 1848 | 205 | 0.000 |
| | 12 | 84 | 2016 | 157 | 0.000 |
| | 14 | 98 | 2352 | 148 | 0.000 |
| | 16 | 112 | 2688 | 182 | 0.005 |
| | 18 | 126 | 3024 | 72 | 0.000 |
| | 20 | 140 | 3360 | 211 | 0.000 |
| | 22 | 154 | 3696 | 43 | 0.030 |
| | 26 | 182 | 4368 | 42 | 0.792 |
| Equation 1 $C_{max\ 1d-28d}/C_{max\ 0-1d}$ | | | | 0.04 | - |

Table 4 is for pK and PD values for Comparative Example 1, and relates to a sustained-release injection product composition capable of continuously releasing deslorelin for 6 months. In order to derive the value according to Equation 1 above, the maximum blood concentration of deslorelin for 1 to 28 days is 697 pg/ml, and the maximum blood concentration of deslorelin within 1 day is 17,624 pg/ml. It can be confirmed that the value according to Equation 1 is 0.04, which is not included within the range of the present disclosure.

Further, it was confirmed that the time point at which the testosterone inhibitory effect appeared as a PD value of 0.4 ng/ml or less started to appear from the time point when 2 weeks had elapsed. However, the testosterone value increased again after 26 weeks so that it was confirmed that the continuous testosterone inhibitory effect did not appear compared to Example 1.

The results of analyzing pharmacokinetic properties for Example 1 and Comparative Example 1 above are summarized as follows.

[Table 5]

|  | Example 1 | Comparative Example 1 |
|---|---|---|
| Cmax(pg/ml) | 605 | 17624 |
| $AUC_{last}$(day*pg/ml) | 42,210.905 | 41,459.765 |
| $AUC_{-1week}$(day*pg/ml) | 856.905 | 6,666.265 |

According to the experimental results, the maximum blood concentration of deslorelin was confirmed at a time point of 2 weeks after injection in the case of Example 1, and the maximum blood concentration of deslorelin was confirmed at a time point when 1 hour had elapsed after injection in the case of Comparative Example 1. That is, it can be confirmed that Comparative Example 1 exhibits initial over-release of deslorelin, whereas Example 1 exhibits suppressed initial over-release.

Further, the total AUC was confirmed to be the same level, but Example 1 was confirmed to be at a level of about 12.9% of Comparative Example 1 in the case of AUC within 1 week so that it was confirmed that there was a large difference in the amount exposed to deslorelin within 1 week after injection.

Experimental Example 3

Evaluation 2 of Pharmacokinetic Properties

Pharmacokinetic evaluation for Example 2 and Comparative Example 2 of the present disclosure was confirmed.

For evaluation, Example 2 and Comparative Example 2 were administered to a beagle dog, and blood was collected to measure the blood concentration of deslorelin (PK) and the blood concentration of testosterone (PD).

The injection product comprising the microparticles of Preparation Example 1 and Preparation Example 2 used as a sustained-release formulation for 12 months or more contained 11.75 mg of deslorelin.

For the experiment, Example 2 and Comparative Example 2 were administered to each of 5 beagle dogs, and a subcutaneous injection method was used as the route of administration.

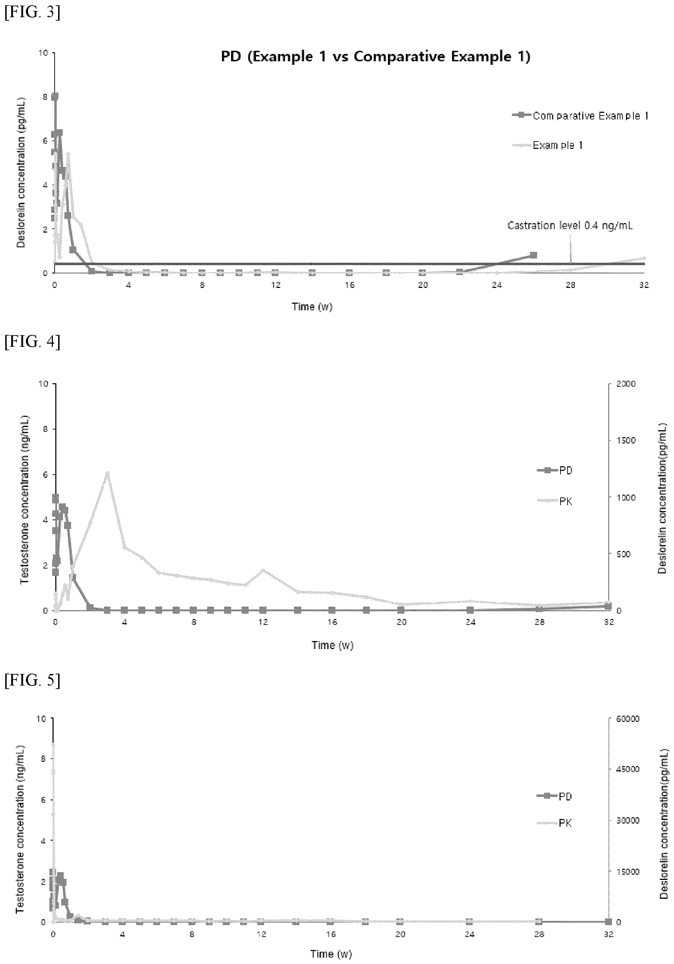

After blood collection, the mean values of PK and PD for 5 beagle dogs were calculated. The experimental results are as shown in Table 6, Table 7, and FIGS. 4 to 6 below.

[Table 6]

|  | Time (w) | Time (d) | Time (h) | pk (pg/ml) | PD (ng/ml) |
|---|---|---|---|---|---|
|  | 0.000 | 0 | 0 | 0 | 2.044 |
|  | 0.001 | 0.01 | 0.25 | 9 | 1.643 |
|  | 0.003 | 0.02 | 0.5 | 41 | 1.685 |
|  | 0.006 | 0.04 | 1 | 138 | 4.264 |
|  | 0.012 | 0.08 | 2 | **146** | 4.841 |

(continued)

|  | Time (w) | Time (d) | Time (h) | pk (pg/ml) | PD (ng/ml) |
|---|---|---|---|---|---|
|  | 0.018 | 0.13 | 3 | 99 | 4.957 |
|  | 0.036 | 0.25 | 6 | 34 | 3.504 |
|  | 0.071 | 0.5 | 12 | 0 | 2.291 |
|  | **0.143** | **1** | **24** | 0 | 2.171 |
|  | 0.286 | 2 | 48 | 53 | 4.112 |
|  | 0.429 | 3 | 72 | 142 | 4.572 |
|  | 0.571 | 4 | 96 | 221 | 4.399 |
|  | 0.714 | 5 | 120 | 106 | 3.708 |
|  | 1 | 7 | 168 | 392 | 1.425 |
|  | 2 | 14 | 336 | 777 | 0.080 |
|  | 3 | 21 | 504 | **1213** | 0.000 |
|  | **4** | **28** | **672** | 553 | 0.000 |
|  | 5 | 35 | 840 | 466 | 0.000 |
| Example 2 | 6 | 42 | 1008 | 326 | 0.000 |
|  | 7 | 49 | 1176 | 309 | 0.000 |
|  | 8 | 56 | 1344 | 280 | 0.000 |
|  | 9 | 63 | 1512 | 268 | 0.000 |
|  | 10 | 70 | 1680 | 237 | 0.000 |
|  | 11 | 77 | 1848 | 224 | 0.000 |
|  | 12 | 84 | 2016 | 354 | 0.000 |
|  | 14 | 98 | 2352 | 167 | 0.000 |
|  | 16 | 112 | 2688 | 152 | 0.000 |
|  | 18 | 126 | 3024 | 115 | 0.000 |
|  | 20 | 140 | 3360 | 50 | 0.000 |
|  | 24 | 168 | 4032 | 76 | 0.000 |
|  | 28 | 196 | 4704 | 44 | 0.053 |
|  | 32 | 224 | 5376 | 69 | 0.176 |
| Equation 1 $C_{max\ 1d\text{-}28d}/C_{max\ 0\text{-}1d}$ |  |  |  | 8.31 | - |

Table 6 above is for pK and PD values for Example 2, and relates to a sustained-release injection product composition capable of continuously releasing deslorelin for 12 months. In order to derive the value according to Equation 1 above, the maximum blood concentration of deslorelin for 1 to 28 days is 1,213 pg/ml, and the maximum blood concentration of deslorelin within 1 day is 146 pg/ml. It can be confirmed that the value according to Equation 1 is 8.31, which is included within the range of the present disclosure.

Further, it was confirmed that the time point at which the testosterone inhibitory effect appeared as a PD value of 0.4 ng/ml or less started to appear from the time point when 1 week had elapsed.

[Table 7]

| | Time (w) | Time (d) | Time (h) | pk (pg/ml) | PD (ng/ml) |
|---|---|---|---|---|---|
| | 0.000 | 0 | 0 | 0 | 1.006 |
| | 0.001 | 0.01 | 0.25 | 31740 | 0.660 |
| | 0.003 | 0.02 | 0.5 | 43592 | 1.658 |
| | 0.006 | 0.04 | 1 | **51975** | 2.050 |
| | 0.012 | 0.08 | 2 | 44417 | 2.017 |
| | 0.018 | 0.13 | 3 | 31674 | 2.437 |
| | 0.036 | 0.25 | 6 | 11323 | 1.610 |
| | 0.071 | 0.5 | 12 | 3170 | 1.247 |
| | **0.143** | **1** | **24** | 1339 | 0.807 |
| | 0.286 | 2 | 48 | 837 | 2.030 |
| | 0.429 | 3 | 72 | 632 | 2.271 |
| | 0.571 | 4 | 96 | 855 | 1.917 |
| | 0.714 | 5 | 120 | 580 | 0.942 |
| **Comparative Example 2** | 1 | 7 | 168 | 476 | 0.255 |
| | 1.4 | 10 | 240 | **1922** | 0.087 |
| | 2 | 14 | 336 | 418 | 0.053 |
| | 3 | 21 | 504 | 557 | 0.005 |
| | **4** | **28** | **672** | 392 | 0.006 |
| | 5 | 35 | 840 | 595 | 0.006 |
| | 6 | 42 | 1008 | 482 | 0.005 |
| | 7 | 49 | 1176 | 403 | 0.004 |
| | 8 | 56 | 1344 | 418 | 0.006 |
| | 9 | 63 | 1512 | 283 | 0.000 |
| | 10 | 70 | 1680 | 401 | 0.010 |
| | 11 | 77 | 1848 | 350 | 0.000 |
| | 12 | 84 | 2016 | 396 | 0.000 |
| | 14 | 98 | 2352 | 385 | 0.005 |
| | 16 | 112 | 2688 | 429 | 0.007 |
| | 18 | 126 | 3024 | 339 | 0.000 |
| | 20 | 140 | 3360 | 325 | 0.005 |
| | 24 | 168 | 4032 | 245 | 0.000 |
| | 28 | 196 | 4704 | 281 | 0.004 |
| Equation 1 $C_{max\ 1d-28d}/C_{max\ 0-1d}$ | | | | 0.04 | |

Table 7 above is for pK and PD values for Comparative Example 2, and relates to a sustained-release injection product composition capable of continuously releasing deslorelin for 12 months. In order to derive the value according to Equation 1 above, the maximum blood concentration of deslorelin for 1 to 28 days is 1,922 pg/ml, and the maximum blood concentration of deslorelin within 1 day is 51,975 pg/ml. It can be confirmed that the value according to Equation 1 is 0.04, which is not included within the range of the present disclosure.

Further, it was confirmed that the time point at which the testosterone inhibitory effect appeared as a PD value of 0.4 ng/ml or less started to appear from the time point when 1 week had elapsed.

When comparing Example 2 and Comparative Example 2 above, it can be confirmed that the testosterone inhibitory effect is equally exhibited for 28 weeks or more. However, Example 2 of the present disclosure is different from Comparative Example 2, which is a conventional product, in that although there is no initial over-release, a long-lasting inhibitory effect of testosterone is exhibited by controlling the release of deslorelin.

The results of analyzing pharmacokinetic properties for Example 2 and Comparative Example 2 above are summarized as follows.

[Table 8]

|  | Example 2 | Comparative Example 2 |
|---|---|---|
| Cmax(pg/ml) | 1,213 | 51,975 |
| $AUC_{last}$(day*pg/ml) | 50,105.12 | 90,005.34 |
| $AUC_{-1week}$(day*pg/ml) | 993.12 | 15,179.34 |

According to the experimental results, the maximum blood concentration of deslorelin was confirmed at a time point of 3 weeks after injection in the case of Example 2, and the maximum blood concentration of deslorelin was confirmed at a time point when 1 hour had elapsed after injection in the case of Comparative Example 2. That is, it can be confirmed that Comparative Example 2 exhibits initial over-release of deslorelin, whereas Example 2 exhibits suppressed initial over-release.

Further, Example 2 was confirmed to be at a level of about 56% of Comparative Example 2 also in the case of total AUC, and Example 2 was confirmed to be at a level of about 12.9% of Comparative Example 2 also in the case of AUC within 1 week so that it was confirmed that there was a large difference in the amount exposed to deslorelin after injection.

Experimental Example 4

Drug Elution Test

Drug elution tests were carried out with respect to Examples 1 to 4 and Comparative Examples 1 and 2.

An elution test solution was prepared by mixing 4 g of PVA and 1 g of tween80 with 1,000 ml of water.

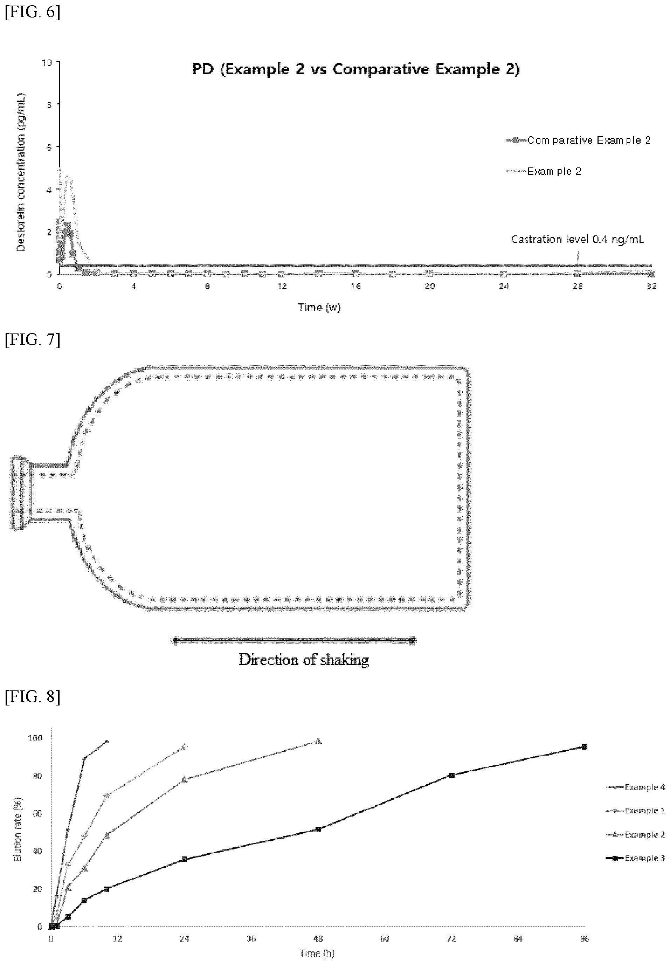

A shaking water bath was used as an elution tester, and a glass test container with an inner capacity of 120ml was used as an elution test container. As shown in FIG. 7, the operating conditions included performing horizontal shaking in the vertical direction to the bottom surface of the container, and performing shaking 150 times (reciprocating) at 55°C.

The experimental results are shown as in FIG. 8 and Table 9.

[Table 9]

| Classification | Elution rate (%) / hour | | | | | | | | |
|---|---|---|---|---|---|---|---|---|---|
|  | 0 | 1 | 3 | 6 | 10 | 24 | 48 | 72 | 96 |
| Example 4 | 0 | 15.9 | 51.4 | 89.2 | 98.3 | - | - | - | - |
| Example 1 | 0 | 5.4 | 32.9 | 48.1 | 69.4 | 95.7 | - | - | - |
| Example 2 | 0 | 0.7 | 20.7 | 31.2 | 48.3 | 78.2 | 98.7 | - | - |
| Example 3 | 0 | 0.3 | 5.1 | 13.9 | 19.8 | 35.7 | 51.5 | 80.4 | 95.6 |
| Comparative Example 1 | 0 | 30.9 | 53.7 | 68.0 | 88.9 | 100.2 | - | - | - |
| Comparative Example 2 | 0 | 22.2 | 40.6 | 59.4 | 78.4 | 95.2 | 100.5 | - | - |

Example 1 and Comparative Example 1 above are both 6-month formulations, and Example 2 and Comparative Example 2 are 12-month formulations. It is confirmed from the results of comparing elution rates that the Examples continuously release deslorelin for a longer period of time than the Comparative Examples.

Although preferred embodiments of the present disclosure have been described in detail above, the right scope of the present disclosure is not limited thereto, and various modifications and improved forms by those skilled in the art using the basic concept of the present disclosure as defined in the following claims are also included in the right scope of the present disclosure.

**Claims**

1. A composition for sustained-release injection comprising deslorelin, the composition comprising microparticles, wherein the microparticles comprise deslorelin and a biodegradable polymer, and a value according to the following Equation 1 is 1 to 10:

[Equation 1]

$$C_{max\ 1d-28d}/C_{max\ 0-1d}$$

where,

the composition for sustained-release injection comprising deslorelin is administered as an injection product to a beagle dog to measure the blood concentration of deslorelin,
$C_{max\ 1d-28d}$ is the maximum blood concentration of deslorelin within 1 to 28 days after injecting the injection product, and
$C_{max\ 0-1d}$ is the maximum blood concentration of deslorelin within 1 day after injecting the injection product.

2. The composition of claim 1, wherein the microparticles have a value according to the following Equation 2 of 1 to 2:

[Equation 2]

$$\frac{D90-D50}{D50-D10}$$

where

D10 is the diameter of the particles corresponding to 10% of the maximum value in the cumulative distribution of particles,
D50 is the diameter of the particles corresponding to 50% of the maximum value in the cumulative distribution of particles, and
D90 is the diameter of the particles corresponding to 90% of the maximum value in the cumulative distribution of particles.

3. The composition of claim 1 or 2, wherein the biodegradable polymer is selected from the group consisting of polylactic acid, polylactide, polylactic-co-glycolic acid, polylactide-co-glycolide (PLGA), polyphosphazines, polyiminocarbonates, polyphosphoesters, polyanhydrides, polyorthoesters, polycaprolactone, polyhydroxyvalate, polyhydroxybutyrate, polyamino acids, and combinations thereof.

4. The composition of any one of claims 1 to 3, wherein the biodegradable polymer has a viscosity of 0.1 to 1.0 dl/g.

5. The composition of any one of claims 1 to 4, wherein deslorelin and the biodegradable polymer are contained at a weight ratio of 1:4 to 1:10.

6. The composition of any one of claims 1 to 5, wherein when administered as an injection product, the release rate of deslorelin at the target site is adjusted, and the inhibitory effect of testosterone due to deslorelin lasts for 1 month or more.

7. The composition of any one of claims 1 to 6, wherein testosterone is maintained at 0.4 ng/ml or less after 10 days have passed since the injection product is administered.

8.  The composition of any one of claims 1 to 7, wherein the composition for injection comprises a suspension solvent.

9.  A method for preparing a composition for sustained-release injection comprising deslorelin, the method comprising the steps of:

    1) preparing a first mixture by mixing deslorelin and a biodegradable polymer;
    2) preparing a second mixture by dissolving a surfactant in a solvent;
    3) injecting the first mixture and the second mixture into a first microchannel and a second microchannel respectively in which an intersection point is formed, and allowing them to flow therethrough to produce microparticles at the intersection point;
    4) collecting the microparticles in a water tank containing the second mixture;
    5) removing an organic solvent present in the collected microparticles;
    6) washing the organic solvent-removed microparticles with purified water and drying the washed microparticles; and
    7) mixing the dried microparticles with a suspension solution, and

    a value according to the following Equation 1 is 1 to 10:

    $$[\text{Equation 1}]$$

    $$C_{max\ 1d\text{-}28d}/C_{max\ 0\text{-}1d}$$

    where,
    the composition for sustained-release injection comprising deslorelin is administered as an injection product to a beagle dog to measure the blood concentration of deslorelin,
    $C_{max\ 1d\text{-}28d}$ is the maximum blood concentration of deslorelin within 1 to 28 days after injecting the injection product, and
    $C_{max\ 0\text{-}1d}$ is the maximum blood concentration of deslorelin within 1 day after injecting the injection product.

10. The method of claim 9, wherein when the first mixture is injected into the first microchannel, after the first mixture is injected under a pressure condition of 700 to 1,500 mbar, the pressure is increased to a first condition of 10 to 30 mbar/min, and when the injection pressure condition reaches 900 to 1,700 mbar, the pressure is increased to a second condition of 2 to 8 mbar/min.

11. The method of claim 9 or 10, wherein the second mixture is injected into the second microchannel under a pressure condition that is 2 to 4 times greater than the pressure condition when the first mixture is injected into the first microchannel.

12. The method of any one of claims 9 to 11, wherein the step 5) includes the steps of: 5-1) performing primary stirring at a speed of 100 to 300 rpm for 20 to 40 minutes at 15 to 20°C; 5-2) performing secondary stirring at a speed of 100 to 300 rpm for 60 to 120 minutes at 30 to 40 °C; and 5-3) performing tertiary stirring at a speed of 100 to 300 rpm for 4 to 8 hours at 40 to 45°C.

[FIG. 1]

[FIG. 2]

[FIG. 3]

PD (Example 1 vs Comparative Example 1)

[FIG. 4]

[FIG. 5]

[FIG. 6]

PD (Example 2 vs Comparative Example 2)

[FIG. 7]

Direction of shaking

[FIG. 8]

Europäisches Patentamt

European Patent Office

Office européen des brevets

# EUROPEAN SEARCH REPORT

Application Number

EP 23 15 6810

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 949 951 A1 (INVENTAGE LAB INC [KR]) 9 February 2022 (2022-02-09) * claims 1-11 * * paragraphs [0055] – [0057] * ----- | 1-12 | INV. A61K9/16 A61K9/10 A61K38/00 A61P5/02 A61K47/38 |
| X | EP 3 900 705 A1 (G2GBIO INC [KR]) 27 October 2021 (2021-10-27) * claims 1-20 * * paragraph [0033] * ----- | 1-12 | |

TECHNICAL FIELDS SEARCHED (IPC)

A61K
A61P

The present search report has been drawn up for all claims

| Place of search | Date of completion of the search | Examiner |
|---|---|---|
| Munich | 4 July 2023 | Vázquez Lantes, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................................
& : member of the same patent family, corresponding document

EPO FORM 1503 03.82 (P04C01)

**EP 4 230 194 A1**

## ANNEX TO THE EUROPEAN SEARCH REPORT
## ON EUROPEAN PATENT APPLICATION NO.

EP 23 15 6810

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2023

| Patent document cited in search report | | Publication date | Patent family member(s) | | Publication date |
|---|---|---|---|---|---|
| EP 3949951 | A1 | 09-02-2022 | AU | 2020266058 A1 | 18-11-2021 |
| | | | CA | 3137819 A1 | 05-11-2020 |
| | | | CL | 2021002761 A1 | 03-06-2022 |
| | | | CN | 113747884 A | 03-12-2021 |
| | | | EP | 3949951 A1 | 09-02-2022 |
| | | | IL | 287361 A | 01-12-2021 |
| | | | JP | 7253287 B2 | 06-04-2023 |
| | | | JP | 2022530878 A | 04-07-2022 |
| | | | KR | 20200126599 A | 09-11-2020 |
| | | | PE | 20220491 A1 | 07-04-2022 |
| | | | SG | 11202111521W A | 29-11-2021 |
| | | | US | 2022202894 A1 | 30-06-2022 |
| | | | WO | 2020222399 A1 | 05-11-2020 |
| | | | ZA | 202108300 B | 25-01-2023 |
| EP 3900705 | A1 | 27-10-2021 | AU | 2019401868 A1 | 22-07-2021 |
| | | | CN | 113473973 A | 01-10-2021 |
| | | | EP | 3900705 A1 | 27-10-2021 |
| | | | JP | 2021534155 A | 09-12-2021 |
| | | | KR | 20200074906 A | 25-06-2020 |
| | | | US | 2022023217 A1 | 27-01-2022 |
| | | | WO | 2020130585 A1 | 25-06-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220021275

- KR 1020120052355 A1

**Non-patent literature cited in the description**

- **THUN R et al.** Castration in male pigs: Techniques and animal welfare issues. *Journal of Physiology and Pharmacology,* 2006, vol. 57, 189-194